# EUROPEAN PATENT SPECIFICATION

(11) **EP 3 823 563 B1**
(45) Date of publication and mention of the grant of the patent: **31.08.2022**
(21) Application number: 19762212.9
(22) Date of filing: 18.07.2019
(51) Int. Cl.: A61F 2/58

(54) **WRIST PROSTHESIS**
HANDGELENKSPROTHESE
PROTHÈSE DE POIGNET

(30) Priority: 20.07.2018 IT 201800007400
(43) Date of publication of application: 26.05.2021
(73) Proprietor: Fondazione Istituto Italiano di Tecnologia, 16163 Genova (IT); Istituto Nazionale per l'Assicurazione Contro gli Infortuni sul Lavoro, 00144 Roma (IT)
(72) Inventor: LINCE, Andrea, 15064 Fresonara (AL) (IT); TRAVERSO, Simone, 16165 Genova (IT); GRUPPIONI, Emanuele, 00144 Roma (IT); LAFFRANCHI, Matteo, 16128 Genova (IT); DE MICHIELI, Lorenzo, 16163 Genova (IT)
(74) Representative: Bottino, Giovanni
(86) International application number: PCT/IB2019/056142
(87) International publication number: WO 2020/016819

(56) References cited:
- EP-A1- 3 102 157
- EP-B1- 3 102 157
- US-A- 4 499 790
- US-A1- 2013 319 160
- US-A1- 2015 122 072

## Description

The present invention relates to a prosthetic wrist unit comprising a drive motor, suitable for being kinematically interposed between the distal end of an arm prosthesis and a prosthetic terminal device, for positioning the terminal device at desired orientations with respect to the arm prosthesis.

The human wrist significantly contributes to hand mobility and handling skills in healthy individuals. The role of the wrist in grasping is to place the hand in the appropriate orientation to allow gripping and handling to be performed. Losing the wrist functionality involves a significant impairment for that person.

However, the prosthetic industry, as well as research, has often neglected the prosthetic wrists in favour of the development of terminal devices.

The design of a prosthetic wrist is the result of compromises between the functionality and practicality of the device. This means that the wrist must be light and compact enough to be carried to the distal end of the arm, regardless of the level of loss. It must also be easy to control in order to allow the bearer to use the device easily, without having to focus on the device and how it should be controlled. Any prosthetic wrist unit increases the mass and cost of the entire limb prosthesis, and there is a perceived lack of improvement in functionality once it has been provided to the user. If the terminal prosthetic device fails to be easily positioned, then it is likely that the user will prefer not to use the prosthetic wrist unit.

Nowadays, it is difficult to be able to realize an active wrist device which is compact enough to fit in the anthropometric dimensions and the socket of the patients.

In addition to this, in order to be used by patients, the mechanism must meet high standards of strength and silence and provide the irreversibility of the system if the user tries to turn the wrist with the engine switched off.

These requirements are not adequately met by the prosthetic wrist units known in the art.

Most commercially available devices are passive, generally devices with 1 or 2 degrees of freedom, having only a few motorized active rotators available.

Most prosthetic wrists with multiple degrees of freedom are mechanisms placed in series, which, although easy to design and manufacture, are more prone to excessive length.

The active prosthetic wrists which can reach driving torques similar to the healthy human wrist can be quite heavy or long.

Document EP3102157A1 describes an arm prosthesis comprising a motorized prosthetic wrist unit comprising a planetary gear transmission, a clutch and a cycloidal transmission. However, the entire prosthesis is heavy and complex.

The relatively compact active prosthetic wrists have a very low driving torque. This compromise must be addressed in the development of more sophisticated wrist prostheses.

Currently, there is thus an unaddressed need for an active wrist prosthesis meeting the high standards of compactness required and capable of ensuring the performance of a human wrist, silent operation and irreversibility of the system with the motor turned off.

The present invention aims at the above-described objectives and at overcoming the drawbacks of the currently known devices with a prosthetic wrist unit as described at the beginning, which further comprises a reduction mechanism formed by a first epicycloidal reduction stage and a second cycloidal reduction stage, connected to each other, wherein the first stage comprises a central wheel connected to the motor shaft, one or more satellite wheels rotatably placed around their own axis on a planet carrier, and a fixed outer ring gear, the satellite wheels being in peripheral contact with the central wheel and with the outer ring gear, and the planet carrier being connected to the second stage, and wherein the central wheel, the satellite wheels and the outer ring gear have smooth mutually engaging surfaces, in such a way that the rolling of the satellite wheels on the outer ring gear is caused by the friction forces generated by radial interference between the central wheel and the satellite wheels and the outer ring gear.

The use of transmission mechanisms is widely known, generally for transferring a motion between an input shaft or element and an output shaft or element through a gear, in various applications, especially for the variation (*i.e.,* multiplication or reduction) of the rotation speed and the driving torque between the input shaft and the output shaft.

More in detail, the gears are gear trains which include typically toothed or lobed members rotatable about respective axes of rotation and cooperating with each other so as to allow the transfer of a rotational motion.

The aforementioned gears define:
- an ordinary gear train when they include a plurality of members in which the axes of rotation are operatively fixed, or
- a planetary gear train when they include at least one planetary member whose axis of rotation is operatively fixed and at least one other satellite member whose axis of rotation is operatively movable.

Among the transmission mechanisms provided with a gear constituting a planetary gear train, epicycloidal transmission mechanisms and cycloidal (or hypocycloidal) transmission mechanisms are known.

The epicycloidal transmission mechanisms comprise a gear which results in a planetary gear train in which a point, belonging to the pitch circle defined by a satellite member suitable for rolling externally in engagement with the pitch circle, defined in turn by a planetary member, operationally traces a trajectory of an epicycloid.

Generally, the epicycloidal transmission mechanisms comprise a plurality of satellite wheels mounted with freedom of rotation on a supporting element referred to as a planet carrier, and a central wheel or sun cooperating externally with the satellite wheels; all this is positioned within a ring, also referred to as a ring gear, bearing a typically toothed or lobed internal engagement surface, with which the satellite wheels cooperate externally.

Typically, such epicycloidal transmission mechanisms are manufactured with traditional gears, in particular with the tooth profile being the involute of a circle.

Such epicycloidal transmission mechanisms have motion reversibility, high precision, reduced mechanical backlash.

By contrast, the cycloidal transmission mechanisms comprise a gear which results in a planetary gear train in which a point, belonging to the pitch circle defined by a toothed or lobed satellite member suitable for rolling internally by eccentric orbital motion in engagement with the pitch circle, defined in turn by a toothed or lobed outer ring gear, operationally traces a trajectory of a hypocycloid.

The cycloidal transmission mechanisms generally have a compact structure, in which the planetary and satellite toothing or lobing have large and solid teeth or lobes, and the reduction ratio equals 1:n, where 1 is an eccentric rotation turn and n is the number of teeth or lobes of the rotating part.

The two-stage configuration of the present invention is characterized by high transmissible torque values, combined with high silence and compactness; the first stage is suitable for low driving torques and high speeds, whereas the second is particularly suitable for being used with high driving torques and low speeds. Furthermore, a characteristic intrinsic to the second cycloidal stage is to ensure a certain degree of irreversibility of the motion, in this case desired, to allow to save the electric power coming from the batteries in the event of continuous static external loads.

In an exemplary embodiment, the first stage is placed proximally and is connected to a motor shaft, and the second stage is placed distally and is connected to a terminal device fastening element.

Therefore, the two mechanisms altogether allow for such a compactness as to fit into the tight dimensions of a wrist prosthesis, and to maintain high structural strength, allowing high mechanical shocks to be absorbed.

The cycloidal mechanism is generally used on machine tools and anthropomorphic industrial robots, such as in shoulder joints, *i.e.,* in applications where a great structural rigidity is required, and weight is not a problem. In this case, such mechanism has been adapted to a small and light version, applicable to reduced diameters specific for the anthropometric size of the wrist, allowing a high reduction in a small space, with high relative transmissible torque.

The balancing and lightening of the two systems allow the creation of an overall mechanism with the necessary performance requirements.

In the first stage, the satellite wheels rotate on the fixed outer ring gear under the action of the central wheel, and transmit the motion to the planet carrier and, accordingly, to the second stage.

Since the central wheel, the satellite wheels and the outer ring gear have smooth mutually engaging surfaces, in such a way that the rolling of the satellite wheels on the ring gear is caused by the friction forces generated by radial interference between the central wheel and the satellite wheels and between the satellite wheels and the outer ring gear, the central wheel can be made up of a bush with a smooth coating surface, keyed onto the motor shaft.

The satellites thus perform a rolling, without sliding, on the fixed outer ring gear due to friction forces. Therefore, no teeth are used for this satellite planetary stage application, as in the usual known applications, but it is preferred to transmit the forces through surface friction, in order to sacrifice part of the transmissible torque and benefit in terms of silence and system costs.

In an advantageous embodiment, the satellite wheels are made up of ball bearings or radial ball bearings with the outer diameter coated in urethane.

However, an internally toothed ring gear and toothed satellite wheels may be provided as an alternative. In this case, the central wheel is made up of a toothed pinion.

In a further exemplary embodiment, the planet carrier is provided with pins for engaging the satellite wheels, the satellite wheels being provided with seats for housing the engagement pins; the housing seats are larger than the engagement pins.

A dimensional gap is thereby formed on the diameter of the engagement pins with respect to the inner diameter of the housing seats provided in the satellite wheels: this is to allow the satellite wheels to adapt automatically to the real size and tolerances of the central wheel and the ring gear, fixing the dimensional / geometric manufacturing mistakes and avoiding to produce hyperstatic mechanical systems which would inevitably lead to breaks.

In a further exemplary embodiment, the second stage comprises an input shaft connected to the first stage and provided with one or more eccentric portions with respect to its own axis, a fixed outer cam defining an inner lobing and centred around the axis of the input shaft, and one or more cycloidal elements. The cycloidal elements are engaged on the eccentric portions of the input shaft so as to be rotatable with eccentric orbital motion with respect to the axis of the input shaft and are provided with a satellite outer lobing meshing peripherally with said inner lobing. The cycloidal elements have a plurality of holes in which a corresponding plurality of protrusions of an output rotating element engages, the output rotating element being connected to the terminal device fastening element.

Therefore, the cycloidal elements, by maintaining the contact on the outer cam, reduce the motion as they have a lower number of lobes than the cam seats made up of the compartments or recesses present in the inner lobing of the outer cam; after a complete revolution, each cycloidal element will have been left behind with respect to the outer cam by the arc of circle equal to the missing compartments, and the speed at which the disks lose ground with respect to the frame is output by the output element to reduce the incoming motion.

In a further exemplary embodiment, the second stage comprises at least two cycloidal elements which are offset from each other with respect to the axis of the input shaft.

The cycloidal elements may be arranged so as to show any phase difference. Preferably, two cycloidal elements that are offset by 180° are provided, which, thanks to their operating symmetry, ensure the dynamic equilibrium of the system, so as to extend the lifetime of the mechanism and its components, decrease noise and vibrations. The dynamic equilibrium is due to the distribution of the contact forces between the cycloidal elements and the outer cam which, due to the 180° offset between the cycloidal elements, are always equal and opposite in any operating point, thus avoiding the generation of unwanted second order vibrations. In order to eliminate other types of vibrations (following orders), additional cycloidal elements may be added, suitably phased with each other. This addition of cycloidal elements allows to transmit a greater output driving torque, as well as to better withstand external impulsive loads, although, on the other hand, increases the weight and length of the prosthetic unit, not to mention a difficulty of assembly.

In a further exemplary embodiment, the outer cam is formed by a plurality of dowels parallel to each other arranged to form a crown around the axis of the input shaft and angularly equally spaced apart so as to form cam seats for engaging with the outer lobing of the cycloidal elements.

In a further exemplary embodiment, the output rotating element comprises two separate parts, a first part of which is placed proximally with respect to the cycloidal elements and a second part is placed distally with respect to the cycloidal elements, the first part and the second part being connected to each other by means of the said protrusions.

This allows an easy assembly of the device and ensures greater resistance to the bending and axial load of the output, without compromising the overall length constraint.

In a further exemplary embodiment, the planet carrier of the first stage is integrally engaged with the input shaft of the second stage by shape coupling.

This configuration ensures high torque transmission with little available surface and high ease of assembly and disassembly.

In a further exemplary embodiment, the second stage has a recess in the central area of the proximal end surface, the recess in the assembled condition housing at least part of the first stage.

This allows a decrease in the total length of the prosthetic unit.

In a further exemplary embodiment, the first and second stages are housed in an outer frame, one or more ball bearings being provided interposed between the output rotating element and the outer frame.

The use of such bearings appropriately placed in linear positions makes it possible to compensate the loads acting from the outside, ensuring greater resistance to the bending load. Alternatively, it is possible to use bushes (or bearings); in this case the bearable load would be even greater, but there would be greater losses due to friction.

In a further exemplary embodiment, the output element is shaped so as to allow integration with the prosthetic terminal device to be connected downstream. The output element can be shaped so as to integrally complete the terminal device fastening element. Alternatively, the output element is fixed to the terminal device fastening element.

This allows a further reduction in weight and overall dimensions.

From the above, it follows that the active wrist prosthesis object of the present invention is able to perform, in the presence of a motor, the human pronation and supination movements, not exceeding the overall dimensions imposed by human anthropomorphism and by the socket of amputated patients, all this respecting the values of torque and speed of a natural human wrist.

These and other features and advantages of the present invention will become clearer from the following description of some non-limiting exemplary embodiments illustrated in the attached drawings in which:
Fig. 1 shows a longitudinal sectional view;
Fig. 2 shows a cross-sectional view of the first epicycloidal stage;
Fig. 3 shows a cross-sectional view of the second cycloidal stage;
Figs. 4 and 5 show two different axonometric views of the assembled prosthetic unit.

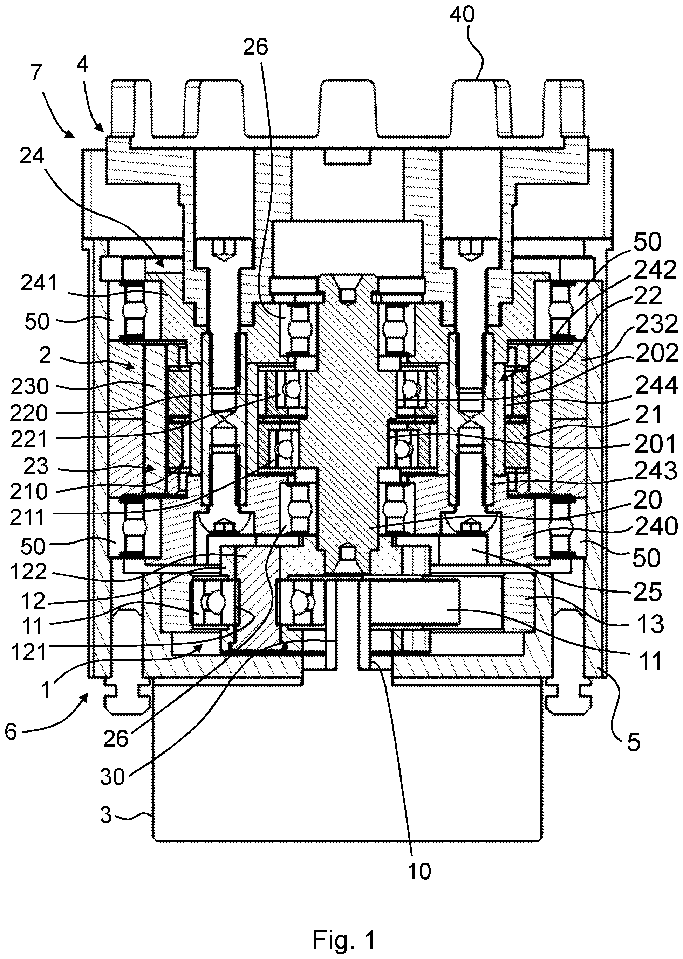

In the sectional view of Figure 1, all the components of the prosthetic wrist unit object of the present invention are visible.

The prosthetic unit is active and therefore comprises a drive motor 3. The motor 3 can be of any currently known type, preferably it is an electric motor, such as a commercial brushless motor. The motor 3 is provided with an output motor shaft 30, which transmits the motion to the downstream kinematic chains. The prosthetic unit is suitable for being kinematically interposed between the distal end of an arm prosthesis and a prosthetic terminal device, such as a hand prosthesis, for positioning the terminal device at desired orientations with respect to the arm prosthesis. The motor 3 is positioned at the proximal end 6 of the prosthetic unit.

At the distal end 7 of the prosthetic unit, *i.e.,* the end for the connection with the prosthetic terminal device, a terminal device fastening element 4 is positioned.

The terminal device fastening element 4 is rotatably actuated by the motor 3 by means of a reduction mechanism, housed within an outer frame 5 made up of a cylindrical casing.

The reduction mechanism comprises a first epicycloidal reduction stage 1 and a second cycloidal reduction stage 2, connected to each other. The first stage 1 is placed proximally and is connected to the motor shaft 30, and is thus suitable for low driving torques and high speeds; the second stage 2 is placed distally and is connected to the terminal device fastening element 4 and is suitable for being used for high driving torques and low speeds.

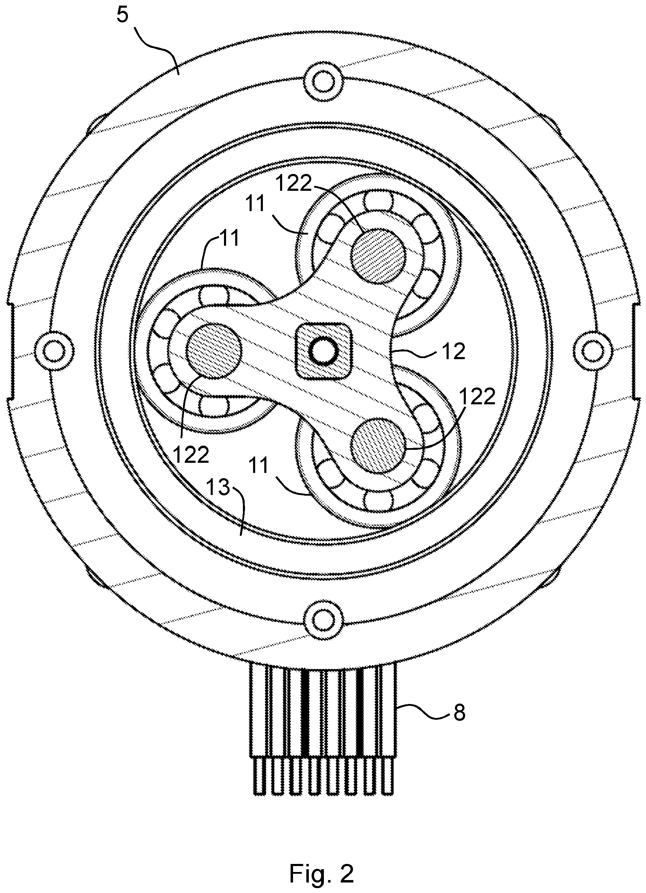

The first stage 1, shown in Figure 2, comprises a central wheel 10 connected to the motor shaft 30, in particular keyed or forced onto the motor shaft 30. In the preferred exemplary embodiment shown in the figure, the central wheel 10 is made up of a bush with a rated outer diameter of 2.95 mm keyed onto the motor shaft 30 and, therefore, has a smooth coating surface.

The first epicycloidal stage 1 further comprises three satellite wheels 11 rotatably placed around its own axis on a planet carrier 12, angularly equally spaced apart from each other by 120°. The satellite wheels 11 are placed in peripheral contact with the central wheel 10 and have smooth engaging surfaces. The planet carrier 12 is provided with engagement pins 122 of the satellite wheels 11 suitable for engaging in corresponding housing seats 121 provided in the satellite wheels 11. The housing seats 121 have a diameter greater than the engagement pins 122 so as to create a dimensional gap to allow the satellite wheels 11 to adapt automatically to the real size and tolerances of the central wheel 10 and of the fixed ring gear 13. The satellite wheels 11 are preferably made up of ball bearings. In this case, the housing seats 122 are made up of the central holes of the ball bearings. In the preferred exemplary embodiment shown in the figure, the satellite wheels 11 have a rated outer diameter of 9 mm and rotate about their axis positioned at a diameter of 12 mm on the planet carrier 12.

The first stage 1 also comprises a fixed outer ring gear 13. The first stage 1 is shaped in such a way that the satellite wheels 11 are in peripheral contact simultaneously with the central wheel 10 and the outer ring gear 13. In the preferred example shown in the figures, the outer ring gear 13 has a rated inner diameter of 20.8 mm. The outer ring gear 13, too, has the engaging surface, *i.e.,* the surface facing inwards, being smooth. The first stage 1 is therefore a roller-based epicycloidal reduction stage, in which the central wheel 10, the satellite wheels 11 and the outer ring gear 13 have smooth mutually engaging surfaces, so that the rolling is caused by the friction forces generated by radial interference between the components.

The entire first stage 1 is implemented through an appropriate design effort on compacting.

The planet carrier 12 represents the output of the first stage 1, being connected to the second stage 2. Therefore, in the first stage 1, the satellite wheels 11 rotate on the fixed outer ring gear 13 under the action of the central wheel 10 and transmit the motion to the planet carrier 12 and, accordingly, to the second stage 2. In the preferred example shown in the figures, the planet carrier 12 consists of two distinct parts joined together by means of the engagement pins 122. However, alternatively, the planet carrier 12 may be provided in a single piece and with engagement pins 122 fixed in a cantilevered manner.

In the preferred exemplary embodiment shown in the figures, the first stage 1 is an epicycloidal mechanism with a reduction ratio of about 8:1.

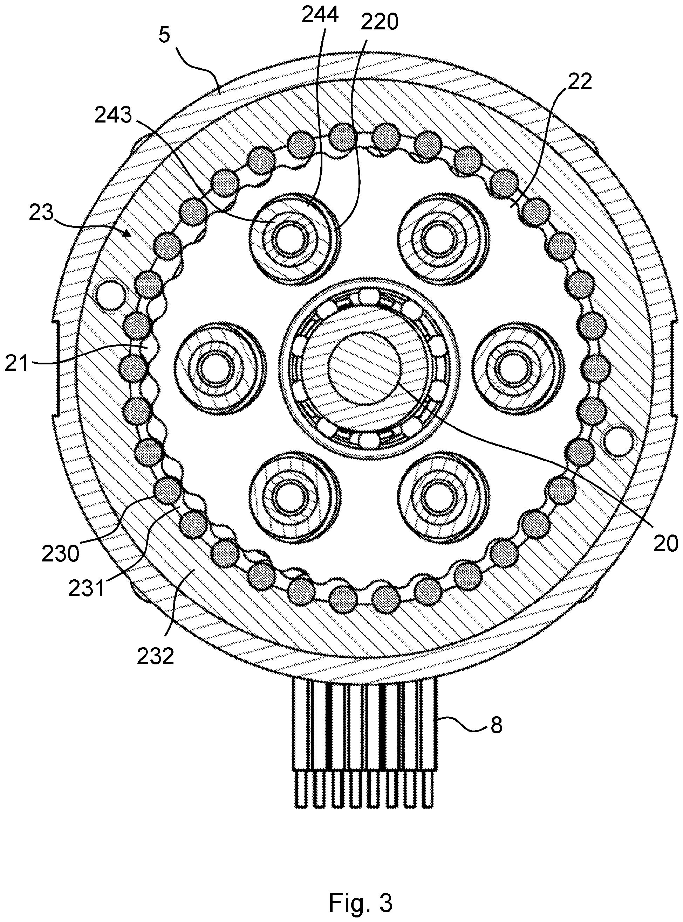

The second cycloidal stage 2, shown in Figure 3, comprises an input shaft 20 connected to the first stage 1. The input shaft 20 is integrally engaged with the planet carrier 12 of the first stage 1 by shape coupling. The input shaft 20 is provided with two eccentric portions with respect to its own axis, a first eccentric portion 201 of which being placed proximally and a second eccentric portion 202 being placed distally and offset by 180 °. The two eccentric portions 201 and 202 are placed adjacent to each other for the sake of compactness, but may also be provided spaced apart.

The second stage 2 further comprises a fixed outer cam 23 defining an inner lobing and centred around the axis of the input shaft 20.

The second stage 2 further comprises two cycloidal elements 21 and 22 in the form of disks. A first cycloidal element 21 is engaged on the first eccentric portion 201 of the input shaft 20 by means of a ball bearing 211, and a second cycloidal element 22 is engaged on the second eccentric portion 202 of the input shaft 20 by means of a ball bearing 221, and they are therefore both rotatable by eccentric orbital motion with respect to the axis of the input shaft 20. The cycloidal elements 21 and 22 are each provided with a satellite outer lobing meshing peripherally with the inner lobing of the outer cam 23.

The cycloidal elements 21 and 22 are offset from each other by 180° with respect to the axis of the input shaft 20, the two eccentric portions 201 and 202 on which they are fixed being offset from each other by a corresponding angle.

The outer cam 23 is formed by a plurality of dowels 230, *e.g.,* of a commercial type, parallel to each other, arranged to form a crown around the axis of the input shaft 20 and angularly equally spaced apart from each other. The dowels 230 are held in place by a supporting fixed outer ring gear 232, integral with the outer frame 5. Compartments or recesses forming cam seats 231 for engaging with the outer lobing of the cycloidal elements 21 and 22 are formed between consecutive dowels 230. The supporting fixed outer ring gear 232 is divided into two parts and is assembled during assembly. Thirty-four dowels 230 are mounted on the supporting fixed outer ring gear 232 and, accordingly, thirty-four cam seats 231 are created between the dowels 230.

The cycloidal elements 21 and 22, in the preferred exemplary embodiment shown in the figure, have a pitch diameter of 24.75 mm and have 33 lobes and 33 compartments, *i.e.,* one less than those of the fixed outer cam 23, to achieve the desired reduction.

The two cycloidal elements 21 and 22, therefore, keeping the contact on the dowels 230 fixed in the supporting outer ring gear 232, reduce the motion as they have one less lobe than the cam seats 231 existing between the dowels 230 of the supporting outer ring gear 232; after a complete revolution, each cycloidal element will have been left behind with respect to the supporting fixed outer ring gear 232 of the arc of circle equal to the missing cam seat 231, and the speed at which the two cycloidal elements 21 and 22 lose ground with respect to the outer frame 5 is output by the output rotating element 24 to reduce the incoming motion impressed by the motor 3.

The two cycloidal elements 21 and 22 have a plurality of holes 210 and 220 placed in a mating position in an assembled condition. A corresponding plurality of protrusions 242 of an output rotating element 24 engages in the holes 210 and 220, being such as to constantly achieve contact during the envelope motion of the cycloidal elements 21 and 22 during their eccentric rotation. The output rotating element 24 is connected to the terminal device fastening element 4. The output rotating element 24 is rotatably engaged with the outer frame 5 by means of two ball bearings 50 and with the input shaft 20 by means of two ball bearings 26.

The output rotating element 24 comprises two separate parts, a first part 240 of which is placed proximally with respect to the cycloidal elements 21 and 22, and a second part 241 is placed distally with respect to the cycloidal elements 20 and 21. The protrusions 242 consist of six pins 243 which connect the first part 240 and the second part 241 to each other, each pin 243 being covered by a bush 244 with reduced rolling resistance friction. Such bushes 244 have a diameter of 4.5 mm, whereas the holes 210 and 220 in the cycloidal elements 21 and 22 have a diameter of 5.04 mm, thereby considering the 0.26 mm eccentricity.

In the preferred exemplary embodiment shown in the figure, the second stage 2 is a cycloidal mechanism with a reduction ratio of 33:1.

Therefore, in the preferred exemplary embodiment shown in the figure, the reduction mechanism of the prosthetic unit has a total reduction ratio of about 264:1.

The second stage 2 has a recess 25 in the central area of the proximal end surface, in particular obtained by suitably shaping the first part 240 of the output rotating element 24. Such recess 25 in the assembled condition of the first stage 1 and of the second stage 2 houses part of the first stage 1, in particular part of the planet carrier 12.

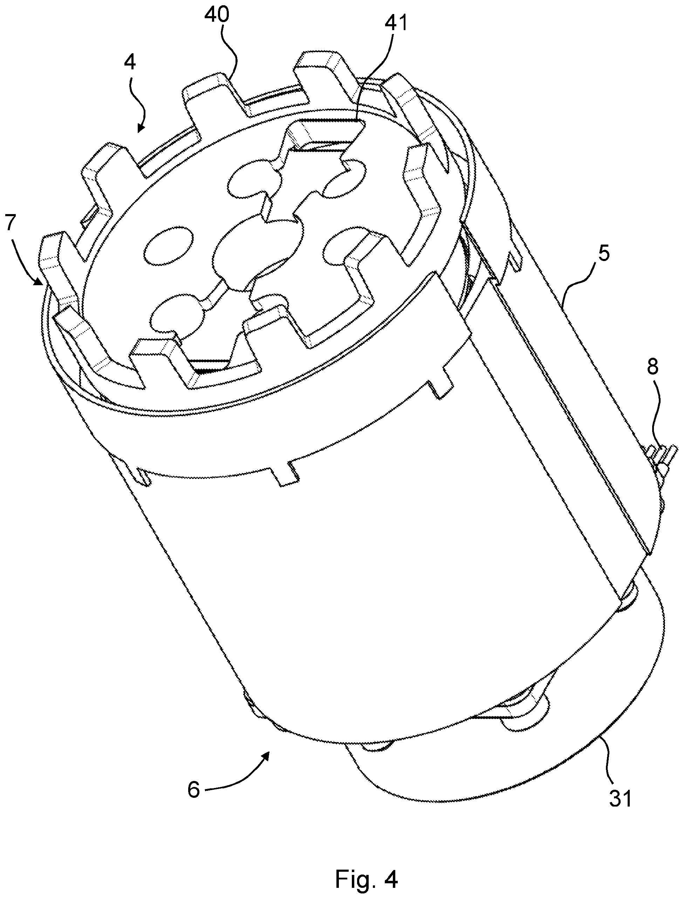

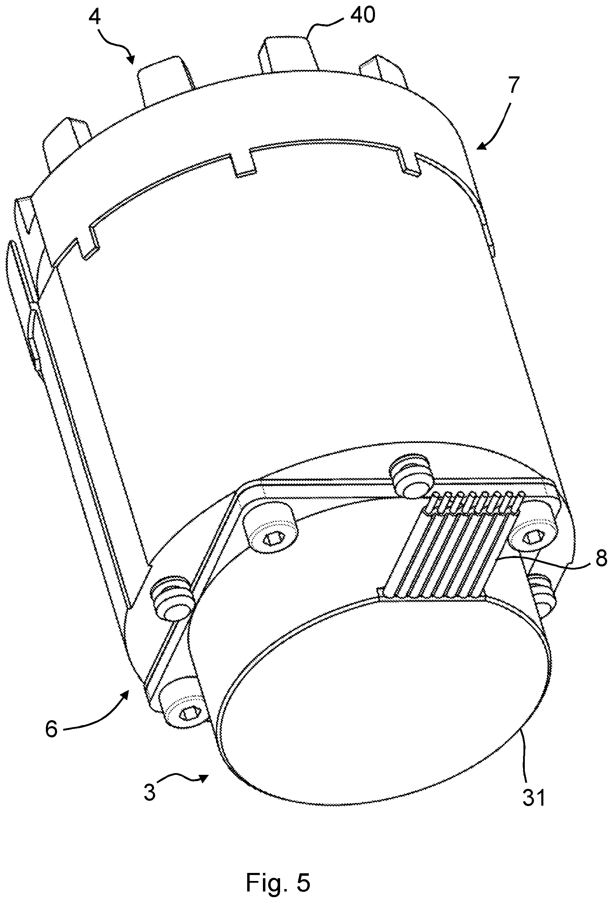

Figures 4 and 5 show two different axonometric views of the assembled prosthetic unit, wherein the cylindrical frame 5 surrounding the first stage 1 and the second stage 2 is visible.

The terminal device fastening element 4 has an end nut 40 provided with engagement teeth suitable for connection with the terminal device. Advantageously, the terminal device fastening element 4 is fixed to the second part 241 of the output rotating element 24 by means of through screws which engage with corresponding threaded seats provided in the pins 243, as shown in Figure 1. The terminal device fastening element 4 also has slots 41 for the passage of control and supply electrical wires.

The motor 3 is enclosed by a cover 31, which can be fixed to the frame 5 by means of screws. The cover 31 is provided with a plurality of electrical terminals 8 for connecting the motor 3 to a supply unit and / or a control unit, not shown in the figures.

The reduction mechanism may comprise further reduction stages and may be varied to adapt the reduction required for other application cases.

Weights may be optimized by using the so-called technopolymer materials.

## Claims

1. A prosthetic wrist unit comprising a drive motor (3), suitable for being kinematically interposed between the distal end of an arm prosthesis and a prosthetic terminal device, for positioning the terminal device at desired orientations with respect to the arm prosthesis, whereby the wrist unit comprises a reduction mechanism comprising a first epicycloidal reduction stage (1) and a second cycloidal reduction stage (2), connected to each other, wherein the first stage (1) comprises a central wheel (10) connected to the motor shaft (30), one or more satellite wheels (11) rotatably placed around their own axis on a planet carrier (12), and a fixed outer ring gear (13), the satellite wheels (11) being in peripheral contact with the central wheel (10) and with the outer ring gear (13), and the planet carrier (12) being connected to the second stage, and **characterized in that** the central wheel (10), the satellite wheels (11) and the outer ring gear (13) have smooth mutually engaging surfaces, in such a way that the rolling of the satellite wheels (11) on the outer ring gear (13) is caused by the friction forces generated by radial interference between the central wheel (10) and the satellite wheels (11) and the outer ring gear (13).

2. The unit according to claim 1, wherein the first stage (1) is placed proximally and is connected to a motor shaft (30), and the second stage (2) is placed distally and is connected to a terminal device fastening element (4).

3. The unit according to claim 1 or 2, wherein the planet carrier (12) is provided with pins (122) for engaging the satellite wheels (11), the satellite wheels (11) being provided with seats (121) for housing the engagement pins (122), the housing seats (121) being larger than the engagement pins (122).

4. The unit according to one or more of the preceding claims, wherein the second stage (2) comprises an input shaft (20) connected to the first stage (1) and provided with one or more eccentric portions (201, 202) with respect to its own axis, a fixed outer cam (23) defining an inner lobing and centred around the axis of the input shaft (20), and one or more cycloidal elements (21, 22), the cycloidal elements (21, 22) being engaged on eccentric portions (201, 202) of the input shaft (20) so as to be rotatable by eccentric orbital motion with respect to the axis of the input shaft (20), and being provided with a satellite outer lobing meshing peripherally on said inner lobing of the outer cam (23), and having a plurality of holes (210, 220) in which a corresponding plurality of protrusions (242) of an output rotating element (24) engages, the output rotating element (24) being connected to the terminal device fastening element (4).

5. The unit according to claim 4, wherein the second stage (2) comprises at least two cycloidal elements (21, 22) which are offset from each other with respect to the axis of the input shaft (20).

6. The unit according to claim 4, wherein the outer cam (23) is formed by a plurality of dowels (230) parallel to each other arranged to form a crown around the axis of the input shaft (20) and angularly equally spaced apart so as to form cam seats (231) for engaging with the outer lobing of the cycloidal elements (21, 22).

7. The unit according to claim 4, wherein the output rotating element (24) comprises two separate parts, a first part (240) of which is placed proximally with respect to the cycloidal elements (21, 22), and a second part (241) is placed distally with respect to the cycloidal elements (21, 22), the first part (240) and the second part (241) being connected to each other by means of the said protrusions (242).

8. The unit according to claim 4, wherein the planet carrier (12) of the first stage (1) is integrally engaged with the input shaft (20) of the second stage (2) by shape coupling.

9. The unit according to one or more of the preceding claims, wherein the second stage (2) has a recess (25) in the central area of the proximal end surface, the recess (25) in the assembled condition housing at least part of the first stage (1).

## Patentansprüche

1. Handgelenkprotheseneinheit, die einen Antriebsmotor (3) für die kinematische Einfügung zwischen dem distalen Ende einer Armprothese und einer prothetischen Endvorrichtung umfasst, zur Positionierung der Endvorrichtung in der gewünschten Ausrichtung bezüglich der Armprothese, wobei die Handgelenkprotheseneinheit
einen Reduktionsmechanismus umfasst, der eine erste epizykloidische Reduktionsstufe (1) und eine zweite zykloidische Reduktionsstufe (2) umfasst, welche miteinander in Verbindung stehen, wobei die erste Stufe (1) ein zentrales Rad (10) umfasst, das mit der Motorwelle (30) in Verbindung steht, ein oder mehrere Satellitenräder (11), die drehbar um ihre eigene Achse auf einem Fahrwerkträger (12) angeordnet sind, und einen festen äußeren Zahnkranz (13), wobei die Satellitenräder (11) in Umfangskontakt mit dem zentralen Rad (10) und mit dem äußeren Zahnkranz (13) stehen und der Fahrradträger (12) mit der zweiten Stufe verbunden ist, und **gekennzeichnet dadurch, dass**
das zentrale Rad (10), die Satellitenräder (11) und der äußere Zahnkranz (13) glatte, ineinander greifende Oberflächen aufweisen, so dass das Abrollen der Satellitenräder (11) auf dem äußeren Zahnkranz (13) von den Reibungskräften erzeugt wird, die durch die radiale Interferenz zwischen dem zentralen Rad (10) und den Satellitenrädern (11) und dem äußeren Zahnkranz (13) entstehen.

2. Einheit nach Anspruch 1, wobei die erste Stufe (1) proximal angeordnet und mit einer Motorwelle (30) verbunden ist und die zweite Stufe (2) distal angeordnet und mit einem Befestigungselement der Endvorrichtung (4) verbunden ist.

3. Einheit nach Anspruch 1 oder 2, wobei der Fahrradträger (12) mit Stiften (122) für den Eingriff mit den Satellitenrädern (11) versehen ist, wobei die Satellitenräder (11) mit Gehäuse-Sitzen (121) zur Aufnahme der Eingriffsstifte (122) versehen sind, wobei die Gehäuse-Sitze (121) größer als die Eingriffsstifte (122) sind.

4. Die Einheit nach einem oder mehreren der vorhergehenden Ansprüche, wobei die zweite Stufe (2) eine Input-Welle (20) umfasst, die mit der ersten Stufe (1) verbunden ist und mit einem oder mehreren exzentrischen Abschnitten (201, 202) bezüglich ihrer eigenen Achse versehen ist, eine feste äußere Nocke (23), die eine innere Aushöhlung bildet und um die Achse der Input-Welle (20) zentriert ist, und ein oder mehrere zykloidische Elemente (21, 22), wobei die zykloidischen Elemente (21, 22) auf exzentrischen Abschnitten (201, 202) der Input-Welle (20) in Eingriff stehen, so dass sie durch eine exzentrische Umlaufbewegung bezüglich der Achse der Input-Welle (20) drehbar sind, und mit einer äußeren Aushöhlung des Satelliten versehen sind, die am Umfang in die innere Aushöhlung der äußeren Nocke (23) eingreift, und eine Vielzahl von Löchern (210, 220) aufweisen, in die eine entsprechende Vielzahl von Vorsprüngen (242) eines Ausgangsdrehelements (24) eingreift, wobei das Ausgangsdrehelement (24) mit dem Befestigungselement der Endvorrichtung (4) verbunden ist.

5. Die Einheit nach Anspruch 4, wobei die zweite Stufe (2) mindestens zwei zykloidische Elemente (21, 22) umfasst, die bezüglich der Achse der Input-Welle (20) gegeneinander versetzt sind.

6. Einheit nach Anspruch 4, wobei die äußere Nocke (23) durch eine Vielzahl von zueinander parallelen Stiften (230) gebildet wird, die so angeordnet sind, dass sie einen Kranz um die Achse der Input-Welle (20) bilden und winkelmäßig gleichmäßig voneinander beabstandet sind, um Nocken-Sitze (231) für den Eingriff mit den äußeren Aushöhlungen der zykloidischen Elemente (21, 22) zu bilden.

7. Einheit nach Anspruch 4, wobei das Ausgangsdrehelement (24) zwei getrennte Teile umfasst, von denen ein erster Teil (240) proximal bezüglich der Zykloidenelemente (21, 22) und ein zweiter Teil (241) distal bezüglich der Zykloidenelemente (21, 22) angeordnet ist, wobei der erste Teil (240) und der zweite Teil (241) durch die genannten Vorsprünge (242) miteinander verbunden sind.

8. Einheit nach Anspruch 4, bei der der Fahrradträger (12) der ersten Stufe (1) durch Formschluss mit der Input-Welle (20) der zweiten Stufe (2) in Eingriff steht.

9. Die Einheit nach einem oder mehreren der vorhergehenden Ansprüche, wobei die zweite Stufe (2) eine Vertiefung (25) im zentralen Bereich der proximalen Endfläche aufweist, wobei die Vertiefung (25) im zusammengebauten Zustand zumindest einen Teil der ersten Stufe (1) aufnimmt.

## Revendications

1. Unité de poignet prothétique comprenant un moteur d'entraînement (3), apte à être interposé cinématiquement entre l'extrémité distale d'une prothèse de bras et un dispositif terminal prothétique, afin de positionner le dispositif terminal à des orientations souhaitées par rapport à la prothèse de bras, par laquelle l'unité de poignet
comprend un mécanisme de réduction comprenant un premier étage de réduction épicycloïdale (1) et un second étage de réduction cycloïdale (2), reliés l'un à l'autre, dans lequel le premier étage (1) comprend une roue centrale (10) reliée à l'arbre de moteur (30), une ou plusieurs roues satellites (11) placées de manière rotative autour de leur propre axe sur un porte-satellites (12), et une couronne extérieure fixe (13), les roues satellites (11) étant en contact périphérique avec la roue centrale (10) et avec la couronne extérieure (13), et le porte-satellites (12) étant relié au second étage, et **caractérisée en ce que** la roue
centrale (10), les roues satellites (11) et la couronne extérieure (13) présentent des surfaces d'engagement mutuel lisses, de sorte que le roulement des roues satellites (11) sur la couronne extérieure (13) soit provoqué par les forces de frottement générées par une interférence radiale entre la roue centrale (10) et les roues satellites (11) et la couronne extérieure (13).

2. Unité selon la revendication 1, dans laquelle le premier étage (1) est placé de manière proximale et est relié à un arbre de moteur (30), et le second étage (2) est placé de manière distale et est relié à un élément de fixation de dispositif terminal (4).

3. Unité selon la revendication 1 ou 2, dans laquelle le porte-satellites (12) est muni de broches (122) pour engager les roues satellites (11), les roues satellites (11) étant munies de sièges (121) pour loger les broches d'engagement (122), les sièges de logement (121) étant plus grands que les broches d'engagement (122).

4. Unité selon une ou plusieurs des revendications précédentes, dans laquelle le second étage (2) comprend un arbre d'entrée (20) relié au premier étage (1) et muni d'une ou plusieurs parties excentriques (201, 202) par rapport à son propre axe, une came extérieure fixe (23) définissant une découpure intérieure et centrée autour de l'axe de l'arbre d'entrée (20), et un ou plusieurs éléments cycloïdaux (21, 22), les éléments cycloïdaux (21, 22) étant engagés sur des parties excentriques (201, 202) de l'arbre d'entrée (20) de manière à pouvoir tourner à travers un mouvement orbital excentrique par rapport à l'axe de l'arbre d'entrée (20), et étant munis d'une découpure extérieure de satellite s'engrenant de manière périphérique sur ladite découpure intérieure de la came extérieure (23), et ayant une pluralité de trous (210, 220) dans lesquels une pluralité correspondante de protrusions (242) d'un élément rotatif de sortie (24) s'engagent, l'élément rotatif de sortie (24) étant relié à l'élément de fixation de dispositif terminal (4).

5. Unité selon la revendication 4, dans laquelle le second étage (2) comprend au moins deux éléments cycloïdaux (21, 22) qui sont décalés l'un par rapport à l'autre par rapport à l'axe de l'arbre d'entrée (20).

6. Unité selon la revendication 4, dans laquelle la came extérieure (23) est formée d'une pluralité de goujons (230) parallèles les uns aux autres agencés pour former une couronne autour de l'axe de l'arbre d'entrée (20) et angulairement équidistants de manière à former des sièges de came (231) pour l'engagement avec la découpure extérieure des éléments cycloïdaux (21, 22).

7. Unité selon la revendication 4, dans laquelle l'élément rotatif de sortie (24) comprend deux parties séparées, dont une première partie (240) est placée de manière proximale par rapport aux éléments cycloïdaux (21, 22), et une seconde partie (241) est placée de manière distale par rapport aux éléments cycloïdaux (21, 22), la première partie (240) et la seconde partie (241) étant reliées l'une à l'autre au moyen desdites protrusions (242).

8. Unité selon la revendication 4, dans laquelle le porte-satellites (12) du premier étage (1) est engagé d'un seul tenant avec l'arbre d'entrée (20) du second étage (2) par couplage de forme.

9. Unité selon une ou plusieurs des revendications précédentes, dans laquelle le second étage (2) présente un évidement (25) dans la zone centrale de la surface d'extrémité proximale, l'évidement (25) dans l'état assemblé logeant au moins une partie du premier étage (1).
